(19) Europäisches Patentamt
European Patent Office
Office européen des brevets

(11) Publication number: 0 189 266
A2

# (12) EUROPEAN PATENT APPLICATION

(21) Application number: 86300202.8

(22) Date of filing: 14.01.86

(51) Int. Cl.4: **C 07 C 49/84**, C 07 C 49/813, C 07 C 45/46, C 07 C 45/80, C 07 C 45/78, C 07 C 45/81, C 07 C 149/34, C 07 F 9/50, C 07 C 97/10

(30) Priority: 18.01.85 US 692462

(43) Date of publication of application: 30.07.86 Bulletin 86/31

(84) Designated Contracting States: DE FR GB IT NL SE

(71) Applicant: THE DOW CHEMICAL COMPANY, 2030 Dow Center Abbott Road P.O. Box 1967, Midland, MI 48640 (US)

(72) Inventor: Walters, Marlin E., 304 Jansen Drive, West Columbia Texas 77486 (US)

(74) Representative: Burford, Anthony Frederick et al, W.H. Beck, Greener & Co. 7 Stone Buildings Lincoln's Inn, London WC2A 3SZ (GB)

## (54) Haloacetyl derivatives of aromatic hydrocarbons.

(57) New compounds which are 4,4'-bis(trihaloacetyl) derivatives of aromatic compounds have been prepared by reacting an aromatic compound with a haloacetyl halide in the presence of a Friedel-Crafts catalyst, such as $AlCl_3$, in a suitable solvent. The compounds are useful in preparing polycarbonates, polyesters, polyamides, polyketones and polyurethanes.

EP 0 189 266 A2

# HALOACETYL DERIVATIVES OF AROMATIC COMPOUNDS

The present invention concerns 4,4'-bis(trihaloacetyl) derivatives of aromatic compounds, a process for their preparation, and their use as monomers in the preparation of polycarbonates, polyesters, polyamides, polyketones and polyurethanes.

Trichloromethylketones have been known for nearly 100 years. Gautier (Ann. chim., (6) 14, 345 [1888]) first prepared 2',2',2'-trichloroacetophenone by acylation of benzene with trichloroacetylchloride in 1888. Although there have been attempts to improve the results of this reaction (Blitz, H., J. Prakt. Chem., (2) 142 193 [1935]; Kaluszyner, A. and Reuter, S., J. Am. Chem. Soc., 75, 5126 [1953]) they have met with little success. Alternative methods for preparing trichloromethylketones have been reported which have the advantage of improved yields such as Houben's (Ber., 59B, 2878 [1926]) nitrile reaction and more recently the oxidation of the corresponding secondary alcohols (Shono, T., Kise, N., Yamazaki, A., and Ohmizu, H., Tetrahedron Lett., 23, 1609 [1982]; Atkins, P.J., Gold, V., and Wassef, W. N., J. Chem. Soc., Chem. Commun., 283 [1983]). Although the latter methods show some improvement, the yields, in general, are less than 50 percent.

The present invention employs a much improved process for the preparation of trihalomethyl ketones from trihaloacetylchloride and an aromatic compound. This invention provides novel trihaloacetyl compounds and more particularly aryl bis (trihaloacetyl) derivatives having the formula:

$$X_3C-\overset{O}{\overset{\|}{C}}-B_1-A-B_2-\overset{O}{\overset{\|}{C}}-CX_3 \qquad (I)$$

wherein $B_1$ and $B_2$ are each independently

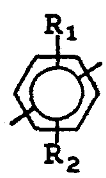 , 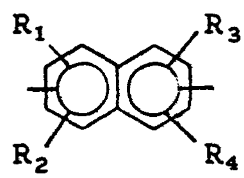 or 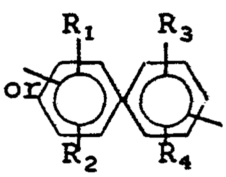 ;

wherein $R_1$, $R_2$, $R_3$ and $R_4$ are each independently hydrogen, chlorine, bromine, an alkyl or an alkoxy group having from 1 to 4 carbon atoms, a phenyl group or

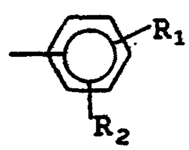

wherein $R_1$ and $R_2$ have their previous meaning;

X is chlorine or bromine; and

A is a single valence bond, oxygen, sulfur,

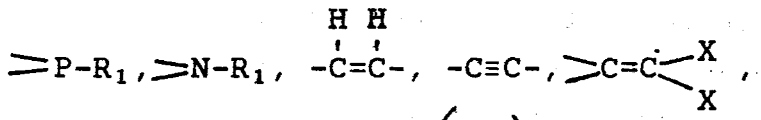

$-CH_2-O-CH_2-$, $-O-CH_2-CH_2-O-$, 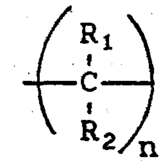 ,

an aromatic group having the formula

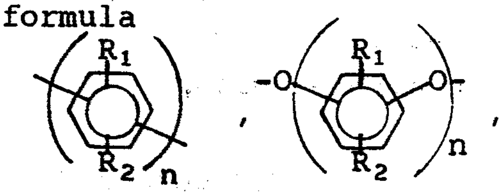

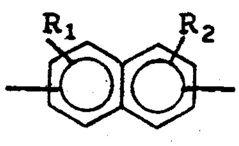 , 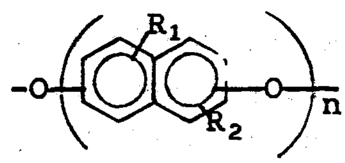 , or

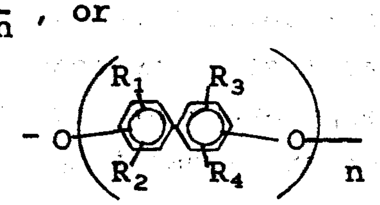

where X, $R_1$, $R_2$, $R_3$ and $R_4$ have their previous meanings and wherein n is an integer from 1 to 6.

Although U.S. Patent 3,150,187 claims aryl dihalogenacetyl derivatives as useful materials which inhibit the growth of <u>Mycobacterium</u> <u>tuberculosis</u>, no mention is made of the trihalogenacetyl derivatives or of any use of them.

The present invention involves the reaction of an aromatic compound with a haloacetyl halide in the presence of a Friedel-Crafts catalyst to prepare the novel compounds of Formula I. Thus, novel compounds of Formula I,which are 4,4'-bis(trihaloacetyl) derivatives of aromatic compounds,have been prepared by reacting an aromatic compound with a haloacetyl halide in the presence of a Friedel-Crafts catalyst, such as $AlCl_3$, in

a suitable solvent. The compounds of Formula I are useful in preparing polycarbonates, polyesters, polyamides, polyketones and polyurethanes. The process for preparing the polymerized products involves reacting the present compounds of Formula I with an organic compound containing at least one hydroxyl, amino, or acryl group and the reactant molecules contain at least two of the reacting functional groups.

The compounds of Formula I are prepared by reacting an aromatic compound with trichloroacetylchloride, tribromoacetylbromide and the like trihaloacetyl halides in a solvent and in the presence of a Friedel-Crafts catalyst. The reaction is preferably conducted at a temperature of from about 20° to about 45°C but may be run at higher temperatures of up to about 85°C. Temperatures above the boiling point of the solvent employed require the use of pressure.

Solvents useful in the process of the invention usually have boiling points of from about 20° to about 135°C and include methylene chloride, perchloroethylene, dichloroethanes, carbon tetrachloride, carbon disulfide, trichloroacetylchloride, halobenzenes, nitroalkanes and other saturated hydrocarbons and their halogenated derivatives having appropriate boiling points providing there is no adverse reaction with the catalyst which might deplete or destroy it.

Useful catalysts for the reaction include $FeCl_3$, $AlCl_3$, $TiCl_3$, $TiCl_4$, $ZrCl_4$, $HfCl_4$, $TaF_5$ and combinations thereof. Aluminum chloride is the preferred catalyst.

The amount of catalyst employed is from about 0.5 to about 4 moles per mole of aromatic compound, but preferably is about 2 to 3. More than about 3 moles of catalyst per mole of aromatic compound provides no advantage.

Pressure normally employed is at or near atmospheric, but if necessary to prevent evaporation of the solvent, pressures up to 10 atmospheres* may be employed. Atmospheric pressure, however, is preferred.

Mole ratios of reactants employed can be from about 2 to about 12 moles of trihaloacetylhalide per mole of aromatic compound. The preferred ratio is from about 2/1 to about 4/1.

The time of reaction is dependent on the temperature and catalyst employed, but is generally from about 0.5 to about 24 hours and preferably from about 2 to about 6 hours.

Representative compounds suitable for acylation with trihaloacetylhalide are, for example, biphenyl, phenyl ether, diphenyl methane, diphenyl ethane, diphenyl propane, diphenyl butane, diphenyl ethylene, diphenyldichloromethane, 1,1,1-trichloro--2,2-bis(phenyl)ethane, 1,1-dichloro-2,2-bis(phenyl)-ethane, 1-chloro-2,2-bis(phenyl)ethane, terphenyl, diphenyl sulfide, diphenylamine, diphenylbenzidine, diphenylmethoxyphosphine, diphenylethoxyphosphine, triphenylphosphine, diphenylfulvene, diphenylphosphine, diphenylmethylsilane, diphenyldimethylsilane, diphenylsilane, diphenoxybenzene, phenoxy biphenyl, diphenoxy biphenyl, 1,4-bis-(4-phenylphenoxy)benzene,

* 1 x $10^3$ kPa

4,4'-bis(4-phenylphenoxy)biphenyl, 1,4-bis(4-phenylphenoxy)naphthalene, 1,5-bis(4-phenylphenoxy)-naphthalene, diphenoxynaphthalene, naphthyl ether, dinaphthoxy benzene, dinaphthoxynaphthylene, diphenoxyethane, benzyl ether and 4,4'-diphenoxybiphenyl.

The reaction to make these products is illustrated in the following example.

Example 1

A slurry of $AlCl_3$ (2.2 moles, 293.26 g) in $CH_2Cl_2$ (200 ml) was prepared in a flask and trichloroacetylchloride (2.0 moles, 363.66 g, TCAC) was added to the mixture from a dropping funnel. Diphenyl ether (1.0 mole, 170.21 g, DPE) was added dropwise over a two-hour period with vigorous stirring. The temperature of the mixture rose from 23°C to 32°C and remained there throughout the reaction. The reaction mixture was poured over ice and the aqueous and organic phases were separated. The $CH_2Cl_2$ solution was washed with saturated aqueous $NaHCO_3$ (2 x 500 ml) then dried over $MgSO_4$, filtered and the solvent was removed by evaporation at reduced pressure, leaving the desired 4,4'-bis-(trichloroacetyl)phenyl ether, which has the following structure:

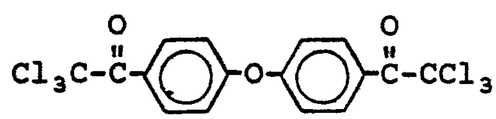

The white crystals had a melting point of 129-130°C and had a yield of 194.8 g or 42.3 percent of theory.

The product can be recrystallized, for example, by dissolving the crystals in methylene chloride and then extracting impurities (mainly color bodies) by contacting with concentrated $H_2SO_4$.

The following example illustrates the use of 1,2-dichloroethane (EDC) as the solvent.

Example 2

A slurry of $AlCl_3$ (7.50 moles, 1000 g) was prepared in EDC (1500 ml) and the trichloroacetylchloride (TCAC) (5.00 moles, 909.15 g) was added to the mixture in one portion. The mixture was brought to 40°C and diphenyl ether (2.50 moles, 425.5 g, 396.6 ml), dissolved in EDC (100 ml), added dropwise with vigorous stirring over a 2 hour period. The temperature rose to 48°C and stabilized during the addition. The reaction mixture was poured over ice (3000 g) and extracted with EDC (1000 ml). The product, 4,4'-bis(trichloroacetyl)phenyl ether, crystallized upon concentration and cooling of the solution to yield 943.82g or 82% of theory, M.p. 129-130°C.

In the following example excess reactant TCAC is used as the solvent.

Example 3

A slurry of $AlCl_3$ (0.44 moles, 59.0 g) was made in TCAC (1.46 mole, 265.75 g, 200 ml) and the mixture brought to 30°C. Diphenyl ether (0.20 moles, 34.04 g) dissolved in TCAC (0.44 moles, 80.0 g, 60 ml) was added over a 35 minute period during which time the reaction temperature rose to 40°C. The mixture was

stirred for 6 hours and then worked up as in the previous example yielding 82.92 g (89.9%) of 4,4'-bis(trichloroacetyl)phenyl ether.

Perchloroethylene was also used effectively as a solvent, but gave a yield of about 80 to 85 percent under substantially the same reaction conditions.

The following example illustrates the use of a mixed solvent system for the reaction.

<u>Example 4</u>

This reaction was run exactly as that described in Example 3 except that a mixture composed of 80% TCAC and 20% perchloroethylene by weight was used as the solvent. Thus, 0.44 mole $AlCl_3$ was slurried in 200 ml of the solvent mixture to which 0.20 moles of diphenyl ether in 60 ml of the solvent mixture was added in 25 minutes. The reaction was worked up after 6 hours as in the above examples yielding 83.61 g (90.7%) of 4,4'-bis(trichloroacetyl)phenyl ether.

The following example illustrates an improved method for the purification of the crude products obtained in these reactions.

<u>Example 5</u>

The crude product obtained from several batches from the reaction of trichloroacetylchloride and di-phenyl ether conducted as in one or more of Examples 1-3 above (2613.0 g) was charged into a 12 liter flask equipped with a mechanical stirrer, nitrogen supply and a thermometer. Methylene chloride (8.0 liters) was added followed by sulfuric acid (98%, 1.0 liter) and

the mixture stirred at 35°C for 24 hours. The mixture was transferred to a separatory funnel and the organic solution separated from the sulfuric acid. The organic layer was then filtered through a short column of silica gel (to remove residual $H_2SO_4$) and the solvent removed by evaporation leaving 2556.07 g (97.82%) of snow white crystals which had a very sharp melting point at 130°C.

Examples of reacting other aromatic compounds with trichloroacetylchloride are shown in Examples 6-11 below.

Example 6 1,4-bis-(4'-trichloroacetylphenoxy)benzene

A slurry of $AlCl_3$ (20 g, 0.15 mol) was made in trichloroacetylchloride (81.5 g, 0.45 mol, 50 ml) under argon at 40°C for 45 min. A solution of 1,4-diphenoxybenzene (10.48 g, 0.04 mol) in perchloroethylene (25 ml) was added dropwise over 15 minutes. The brown mixture was stirred at 40°C for 2 hours and poured over ice (300 ml) and stirred for 1 hour. The colorless precipitate was filtered and dried (22 g, 99.5%) and recrystallized from perchloroethylene to give the desired 1,4-bis(p-trichloroacetylphenoxy)benzene as colorless crystals. The crystals had a melting point of 215°-217°C and have the structure

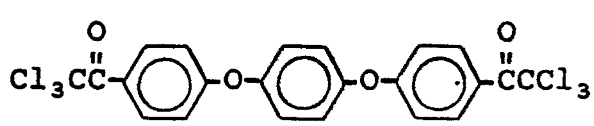

A slightly different preparation of the above compound was made as shown in the following example.

Example 7 - 1,4-Bis(4'-trichloroacetylphenoxy)benzene

To a stirred solution of 1,4-diphenoxybenzene (15.72 g, 0.06 mol) in trichloroacetylchloride (50 ml, 30.69 g, .168 mol) under argon was added $AlCl_3$ (18.5 g, 0.139 mol) all at once. The mixture was stirred and heated at 40°C over 1 hour. After cooling the mixture was poured into 500 g of crushed ice. The resulting colorless crystalline solid was filtered and washed with water. After suction drying overnight the crystals weighed 33 g (99.5% of theory) and had an M.p. of 215°-217°C.

Example 8 - Bis(trichloroacetyl)biphenyl

$AlCl_3$ (80.0 g, 0.60 mol) was weighed into a 500 ml flask which had been flushed with nitrogen. The $AlCl_3$ was then covered with 50 ml of methylene chloride and the TCAC (90.9 g, 0.50 mols) added with stirring. The reaction mixture was brought to 30°C and biphenyl (38.55 g, 0.25 mol) dissolved in $CH_2Cl_2$ (50 ml) was added dropwise over 1 hour. After the mixture had cooled to 25°C the mixture was poured over ice and extracted with $CH_2Cl_2$ (300 ml). The organic layer was washed with water, dried over $MgSO_4$, filtered and concentrated leaving 60.7 g of pale yellow crystals. M.p. 137°C-152°C. GC. Mass Spec. analysis shows the product to be a mixture of isomers wherein the major components were 4,4'-bis(trichloroacetyl)biphenyl and 2,4-bis(trichloroacetyl)biphenyl.

Example 9 - Bis(trichloroacetyl)phenoxybiphenyl

A mixture of TCAC (25 ml), $AlCl_3$ (10.66 g, 0.08 mol) and $CH_2Cl_2$ (40 ml) was stirred at 20°C under argon for 15 minutes. A solution of 4-phenoxybiphenyl

(4.92 g, 0.02 mol) in $CH_2Cl_2$ (30 ml) was added dropwise over 15 minutes and the dark mixture was stirred at 20°C for 4 hours. The usual workup gave a semisolid product which failed to crystallize. GC. Mass Spec. analysis showed this product to be a mixture of isomers of bis(trichloroacetyl)phenoxybiphenyl, wherein the major component is 4-(trichloroacetyl)phenoxy--4'-(trichloroacetyl)biphenyl.

Example 10 - 4,4'-Bis(trichloroacetyl)-3-methyldiphenyl Ether

$AlCl_3$ (200 g, 1.50 mol) was weighed into a 500 ml round bottom flask equipped with mechanical stirrer, thermocouple and a dropping funnel. TCAC (250 ml) was added and the resulting grey suspension stirred for one hour at 23°C. Phenyltolyl ether (92.1 g, 0.05 mol) was then added dropwise from the addition funnel as a solution in 30 ml TCAC over a 60-minute period. The temperature rose to 43°C as the mixture darkened. The mixture was stirred at 45°C for an additional 60 minutes and then poured over ice and extracted with $CH_2Cl_2$. After drying and concentration crystals of 4,4'-bis(trichloroacetyl)-3-methyldiphenyl ether were collected (223.2 g, 94%) M.p. 120°-124°C.

Example 11 - 4,4'-Bis(trichloroacetyl)diphenylmethane

$AlCl_3$ (200 g, 1.50 mol) was weighed into a 500 ml round bottom flask equipped with mechanical stirrer, thermocouple and a dropping funnel. TCAC (250 ml) was added and the resulting grey suspension stirred for 1 hour at 24°C. Diphenylmethane (84.12 g, 0.50 mole) was then added from the dropping funnel dropwise as a solution in 30 ml TCAC over a 45-minute period. The temperature rose from 24°C to 45°C as the diphenylmethane was added. The mixture was stirred at 45°C for an additional 60 minutes and then worked up as usual. The product crystallized from petroleum ether has m.p. 123°-125°C.

0189266

## CLAIMS

1. A compound of the formula:

$$X_3C-\overset{O}{\overset{\|}{C}}-B_1-A-B_2-\overset{O}{\overset{\|}{C}}-CX_3 \qquad (I)$$

wherein $B_1$ and $B_2$ are each independently

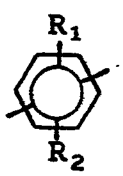 , 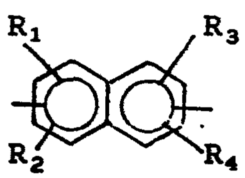 or 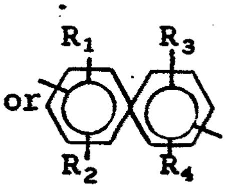 ;

wherein $R_1$, $R_2$, $R_3$ and $R_4$ are each independently hydrogen, chlorine, bromine, an alkyl or an alkoxy group having from 1 to 4 carbon atoms, a phenyl group or

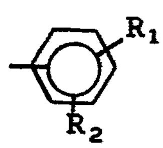

wherein $R_1$ and $R_2$ have their previous meaning;

X is chlorine or bromine; and

A is a single valence bond, oxygen, sulfur,

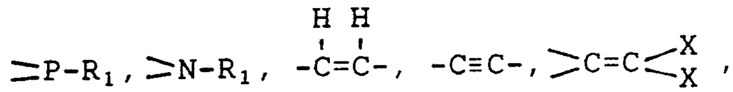

-CH$_2$-O-CH$_2$-, -O-CH$_2$-CH$_2$-O-, 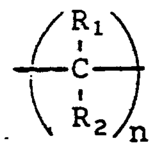 ,

an aromatic group having the formula

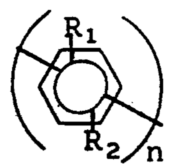 , 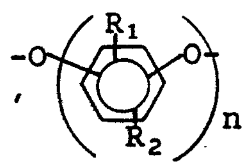 ,

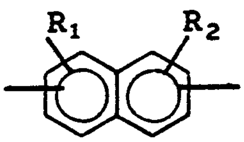 , 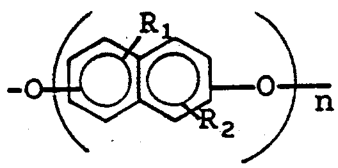 , or

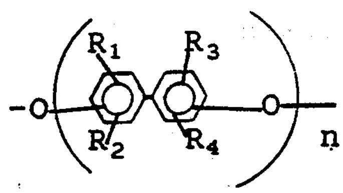

where X, $R_1$, $R_2$, $R_3$ and $R_4$ have their previous meanings and wherein n is an integer from 1 to 6.

2. A compound as claimed in Claim 1, wherein A is oxygen.

3. A compound as claimed in Claim 1, wherein A is a single valence bond.

4. A compound as claimed in Claim 1, wherein A is a methylene group.

5. A compound as claimed in Claim 1, wherein A is

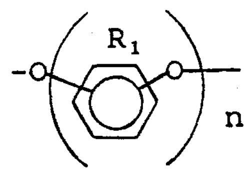

and $R_1$ and n are defined as in Claim 1.

6. A compound as claimed in Claim 1, wherein A is

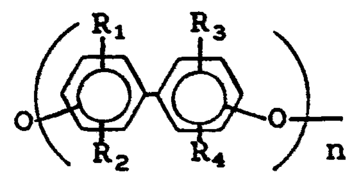

and $R_1$, $R_2$, $R_3$, $R_4$ and n are defined as in Claim 1.

7. A compound as claimed in any one of Claims 1 to 6, wherein $B_1$ and $B_2$ are each

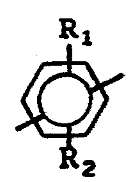

and $R_1$ and $R_2$ are defined as in Claim 1.

8. A compound as claimed in Claim 7, wherein $R_1$ and $R_2$ are each hydrogen on each of $B_1$ and $B_2$.

9. A compound as claimed in Claim 7, wherein at least one of the R groups is an alkyl radical and the remainder are hydrogen.

10. The compound as claimed in Claim 1, which is any one of the following:

4,4'-bis(trichloroacetyl)phenyl ether,

1,4-bis(p-trichloroacetylphenoxy)benzene,

4,4'-bis(trichloroacetyl)biphenyl,

4-(trichloroacetyl)phenoxy--4'-(trichloroacetyl)biphenyl,

4,4'-bis(trichloroacetyl)-3-methyldiphenyl ether, or

4,4'-bis(trichloroacetyl)diphenylmethane.

11. A process for preparing a compound of Formula I as defined in Claim 1 which comprises reacting a trihaloacetyl halide with an aromatic compound in the presence of a Friedel Crafts catalyst in a suitable solvent.

12. A process for purifying an aryl bis(trihaloacetyl) compound of Formula I as defined in Claim 1 which comprises (1) dissolving said compound in a halogenated hydrocarbon solvent, (2) adding concentrated sulfuric acid to the solution of said compound, (3) stirring the solvent and acid mixture for a time sufficient to remove the impurities present in the solvent solution, (4) separating the solvent solution from the acid, (5) treating said solution to remove residual acid, (6) evaporating said solvent and (7) recovering crystals of purified aryl bis(trihaloacetyl) compound.